# EUROPEAN PATENT SPECIFICATION

(11) **EP 3 545 109 B1**
(45) Date of publication and mention of the grant of the patent: **05.01.2022**
(21) Application number: 17825751.5
(22) Date of filing: 27.11.2017
(51) Int. Cl.: C12Q 1/6886

(54) **IN VITRO METHOD FOR IDENTIFYING COLORECTAL ADENOMAS OR COLORECTAL CANCER**
IN-VITRO-VERFAHREN ZUR IDENTIFIZIERUNG KOLOREKTALER ADENOME ODER VON KOLOREKTALEM KREBS
PROCÉDÉ IN VITRO D'IDENTIFICATION D'ADÉNOMES COLORECTAUX OU DU CANCER COLORECTAL

(30) Priority: 28.11.2016 EP 16382567
(43) Date of publication of application: 02.10.2019
(73) Proprietor: Advanced Marker Discovery, S.L. (Amadix), 47004 Valladolid (ES)
(72) Inventor: HERREROS VILLANUEVA, Marta, 47004 Valladolid (ES); MARTÍN RODRÍGUEZ, Ana María Carmen, 47004 Valladolid (ES); PÉREZ PALACIOS, Rosa, 47004 Valladolid (ES); MARTÍNEZ GONZÁLEZ, Miguel Angel, 47004 Valladolid (ES); GIRONELLA COS, Meritxell, 08036 Barcelona (ES); CASTELLS GARANGOU , Antoni, 08036 Barcelona (ES); ARROYO ARRANZ, Rocío, 47004 Valladolid (ES)
(74) Representative: Hoffmann Eitle
(86) International application number: PCT/EP2017/080560
(87) International publication number: WO 2018/096154

(56) References cited:
- CN-B- 102 933 719
- US-A1- 2013 102 487
- ADAM E. FRAMPTON ET AL: "miR-15b and miR-17 Are Tumor-derived Plasma MicroRNAs Dysregulated in Colorectal Neoplasia", ANNALS OF SURGERY., vol. 262, no. 2, 1 August 2015 (2015-08-01), pages e61-e62, XP055368431, US ISSN: 0003-4932, DOI: 10.1097/SLA.0000000000000605
- KANAAN ZIAD ET AL: "A plasma microRNA panel for detection of colorectal adenomas: a step toward more precise screening for colorectal cancer", ANNALS OF SURGERY, J.B. LIPPINCOTT COMPANY, PHILADELPHIA, US, vol. 258, no. 3, 1 September 2013 (2013-09-01), pages 400-408, XP009194236, ISSN: 0003-4932
- Anonimous: "TaqMan Human MicroRNA Arrays", Science Curr Opin Genet Dev, 1 January 2008 (2008-01-01), pages 1-2, XP055368404, Retrieved from the Internet: URL:https://tools.thermofisher.com/content /sfs/manuals/cms_054742.pdf [retrieved on 2017-05-02]
- CHARLES-HENRY GATTOLLIAT ET AL: "MicroRNA and targeted mRNA expression profiling analysis in human colorectal adenomas and adenocarcinomas", EUROPEAN JOURNAL OF CANCER, vol. 51, no. 3, 1 February 2015 (2015-02-01), pages 409-420, XP055368359, AMSTERDAM, NL ISSN: 0959-8049, DOI: 10.1016/j.ejca.2014.12.007
- Anonymous: "The Agilent Human miRNA Microarray", , 18 December 2015 (2015-12-18), pages 1-4, XP055699760, Retrieved from the Internet: URL:https://www.agilent.com/cs/library/bro chures/5989-6226EN_Final_low.pdf [retrieved on 2020-05-29]

## Description

### FIELD OF THE INVENTION

The present invention can be included in the field of personalized medicine, wherein specific biomarkers are used for identifying a given disease or disorder. Specifically, some microRNAs (also named miRNAs or miR-) are used in the present invention for identifying human subjects at risk of developing colorectal cancer (CRC) and/or colorectal adenomas (CA), preferably advanced colorectal adenomas (AA).

### PRIOR ART

Colorectal cancer (also known as colon cancer, rectal cancer, or bowel cancer) is the development of cancer in the colon or rectum (parts of the large intestine). The vast majority of colorectal cancers are adenocarcinomas. This is because the colon has numerous glands within the tissue. When these glands undergo a number of changes at the genetic level, they proceed in a predictable manner as they move from benign to an invasive, malignant colon cancer. The adenomas of the colon, also called adenomatous polyps, are a benign version of the malignant adenocarcinomas but still with malignant potential if not removed (they are usually removed because of their tendency to become malignant and to lead to colon cancer).

Screening is an effective way for preventing and decreasing deaths from colorectal cancer and is recommended starting from the age of 50 to 75. The best known and most frequently used screening test for colorectal cancer is called Fecal Immunochemical Test (FIT). FIT detects blood in the stool samples which can be a sign of pre-cancer or cancer. If abnormal results are obtained, usually a colonoscopy is recommended which allows the physician to look at the inside of the colon and rectum to make a diagnosis. During colonoscopy, small polyps may be removed if found. If a large polyp or tumor is found, a biopsy may be performed to check if it is cancerous. The gastroenterologist uses a colonoscopy to find and remove these adenomas and polyps to prevent them from continuing to acquire genetic changes that will lead to an invasive adenocarcinoma.

Although, as explained above, FIT is nowadays used for screening colorectal cancer, it is important to note that FIT offers a low sensitivity for adenomas (around 20-30% depending on literature) which means that most of said kind of patients can be wrongly classified as not having the disease. Consequently, FIT is not able to identify adenomas due to its low sensitivity.

Moreover, since FIT uses stool samples, it offers a low compliance (less than 50%). On the other hand, the colonoscopy is an invasive technique wherein the most severe complication generally is the gastrointestinal perforation (1% of the cases). Moreover, colonoscopy is nowadays a procedure involving anesthesia, and the laxatives which are usually administered during the bowel preparation for colonoscopy are associated with several digestive problems. ADAM E. FRAMPTON ET AL, ANNALS OF SURGERY. vol. 262, no. 2, 1 August 2015 (2015-08-01), pages e61-e62, refers to miR-15b and miR-17 as being tumor-derived plasma MicroRNAs dysregulated in Colorectal Neoplasia.

The present invention offers a clear solution to the problems cited above because it is focused on an *in vitro* method for identifying or screening human subjects at risk of suffering from colorectal cancer or colorectal adenomas (preferably advanced colorectal adenomas), departing from the level of expression of microRNAs isolated from minimally-invasive samples such as blood, serum or plasma. Moreover, the method of the invention offers high sensitivity and specificity (see the Examples shown below), which means that it is a strong and cost-effective method for the detection of both colorectal cancer or colorectal adenomas (preferably advanced colorectal adenomas).

It is important to note that the methods used today for screening general population at risk of suffering for CRC or AA are associated with a high rate of false positives. Consequently a high amount of unnecessary follow-up colonoscopies are nowadays performed. However, since the method of the invention has higher sensitivity and specificity as compared which the method used today for screening general population at risk of suffering from CRC or AA, it is associated with a lower percentage of false positives. Consequently, the method described in the present invention clearly helps in reducing the number of follow-up colonoscopies, thus improving the way that the patients are nowadays screened or diagnosed. Once the method of the invention is performed, if we see that the miRNAs are overexpressed, the result needs to be confirmed by colonoscopy. However, if we obtain a negative result because the miRNAs are down-regulated, there is no need to perform a colonoscopy and the patients go home.

### BRIEF DESCRIPTION OF THE INVENTION

The present invention is defined by the appended claims. The present invention offers a clear solution for accurately screening and diagnosing subjects at risk of suffering from colorectal cancer and/or colorectal adenomas, by means of a minimally-invasive method able to detect not only patients at risk of suffering from colorectal cancer but also patients which could suffer from colorectal adenomas, preferably advanced colorectal adenomas. In particular, the present disclosure refers to an *in vitro* method for screening for subjects at average risk of developing colorectal adenomas and/or colorectal cancer by measuring the expression pattern or level of at least miR-15b in plasma samples.

### BRIEF DESCRIPTION OF THE FIGURES

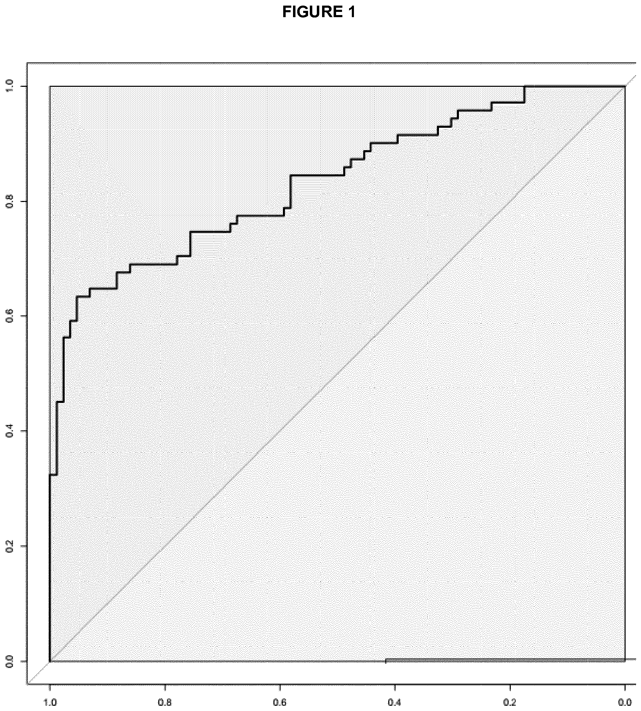

**Fig 1****.** Receiver-operating-characteristic (ROC) curve for miR-15b in colorectal cancer. Area Under Curve (AUC) = 0.833. X axis represents Specificity. Y axis represents Sensitivity.

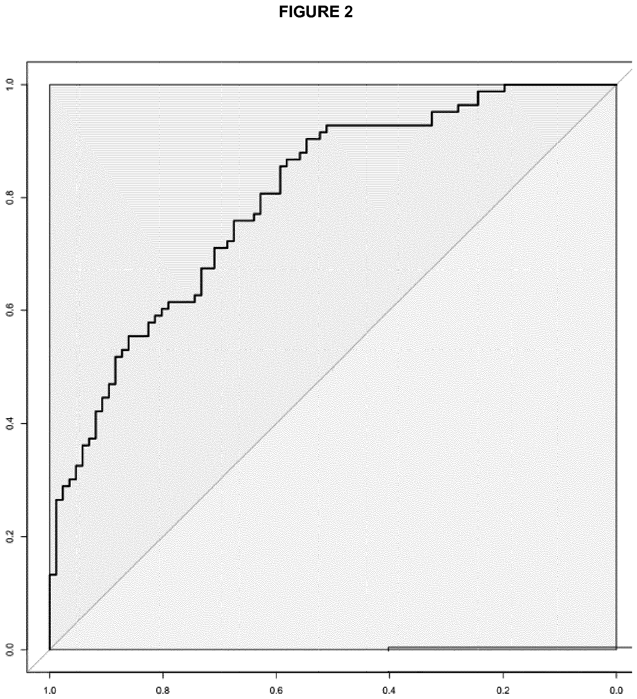

**Fig 2****.** Receiver-operating-characteristic (ROC) curve for miR-15b in colorectal advanced adenoma. Area Under Curve (AUC) = 0.798. X axis represents Specificity. Y axis represents Sensitivity.

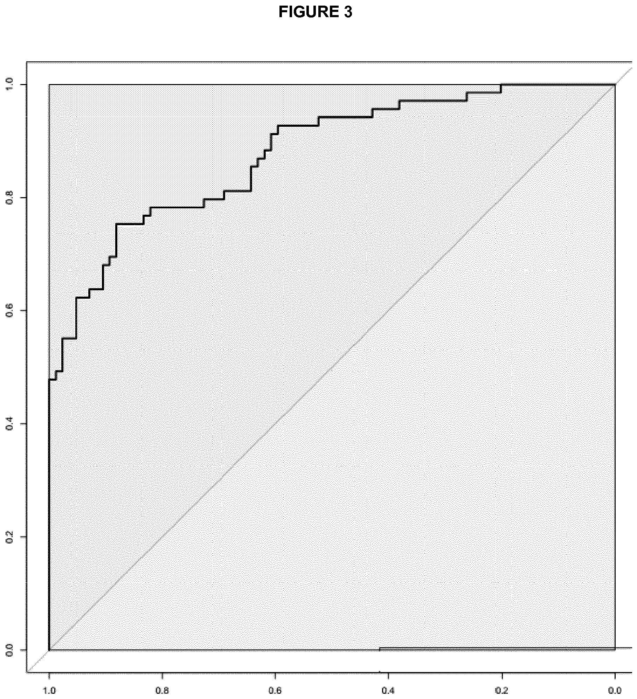

**Fig 3****.** Receiver-operating-characteristic (ROC) curve for miR-29a+miR-15b in colorectal cancer. Area Under Curve (AUC) = 0.878. X axis represents Specificity. Y axis represents Sensitivity.

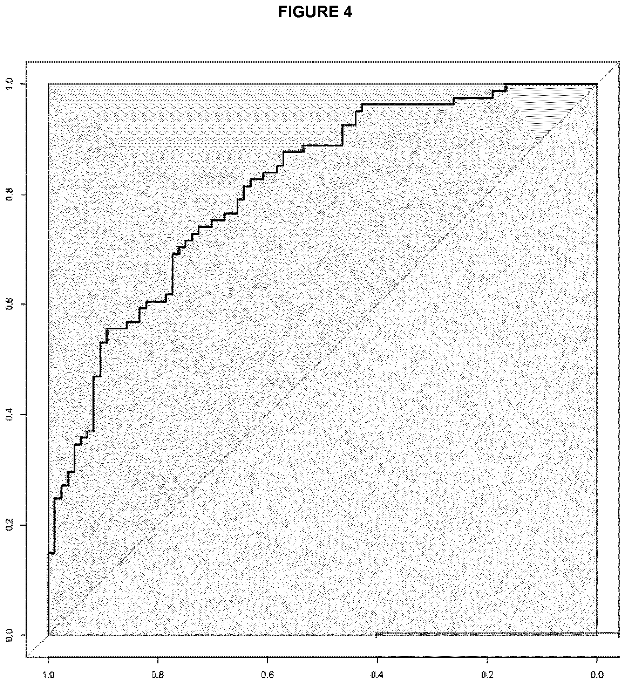

**Fig 4****.** Receiver-operating-characteristic (ROC) curve for miR-29a+miR-15b in colorectal advanced adenoma. Area Under Curve (AUC) = 0.811. X axis represents Specificity. Y axis represents Sensitivity.

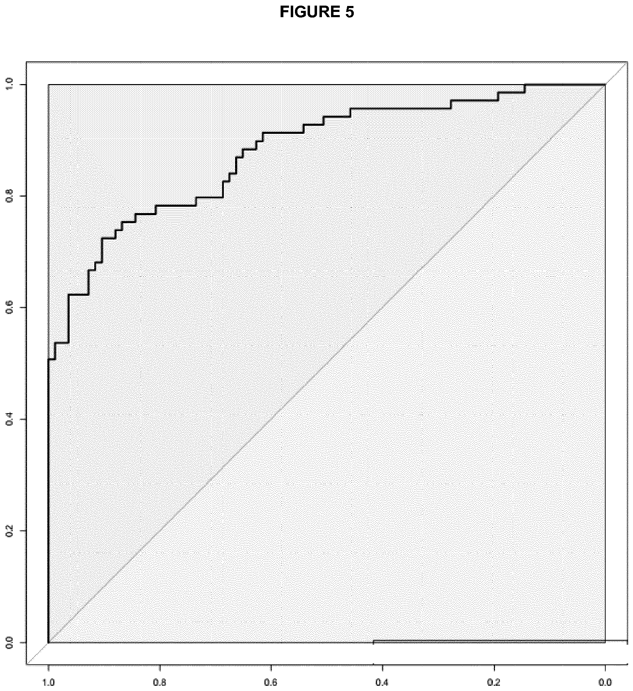

**Fig 5****.** Receiver-operating-characteristic (ROC) curve for miR-29a+ miR-15b+ miR-18a in colorectal cancer. Area Under Curve (AUC) = 0.880. X axis represents Specificity. Y axis represents Sensitivity.

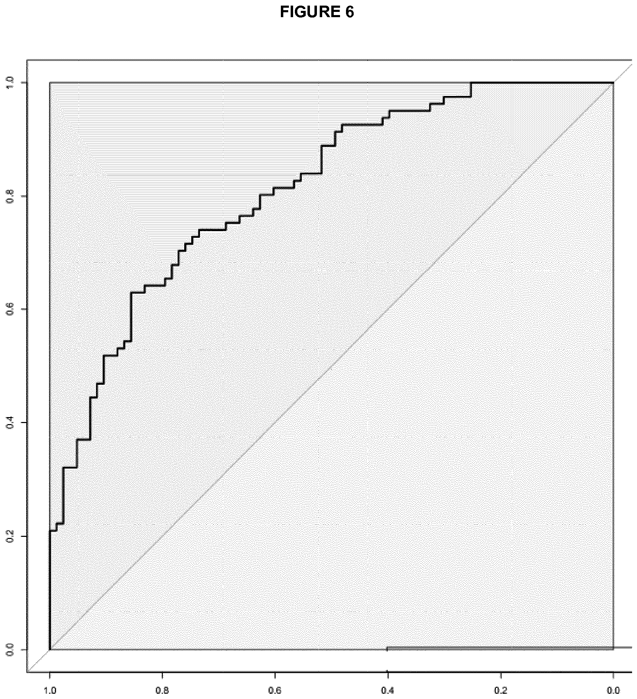

**Fig 6****.** Receiver-operating-characteristic (ROC) curve for miR-29a+ miR-15b+ miR-18a in colorectal advanced adenoma. Area Under Curve (AUC) = 0.812. X axis represents Specificity. Y axis represents Sensitivity.

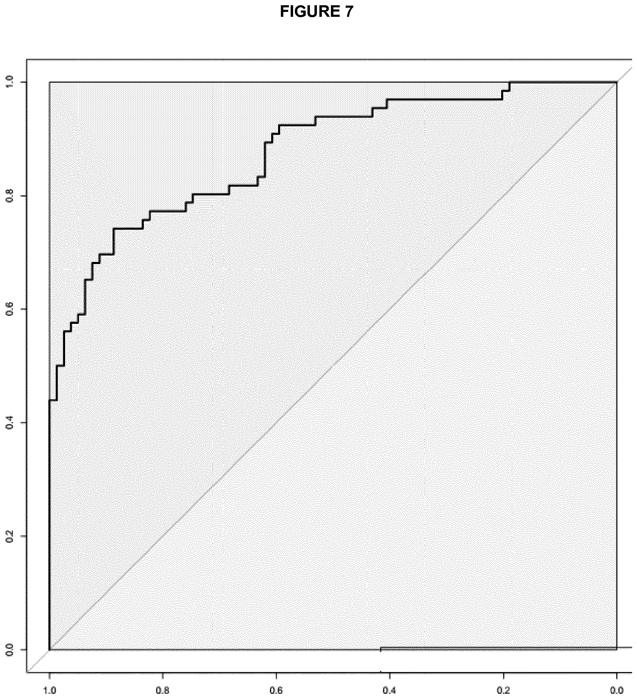

**Fig 7****.** Receiver-operating-characteristic (ROC) curve for miR-29a+ miR-15b+ miR-19b in colorectal cancer. Area Under Curve (AUC) = 0.876. X axis represents Specificity. Y axis represents Sensitivity.

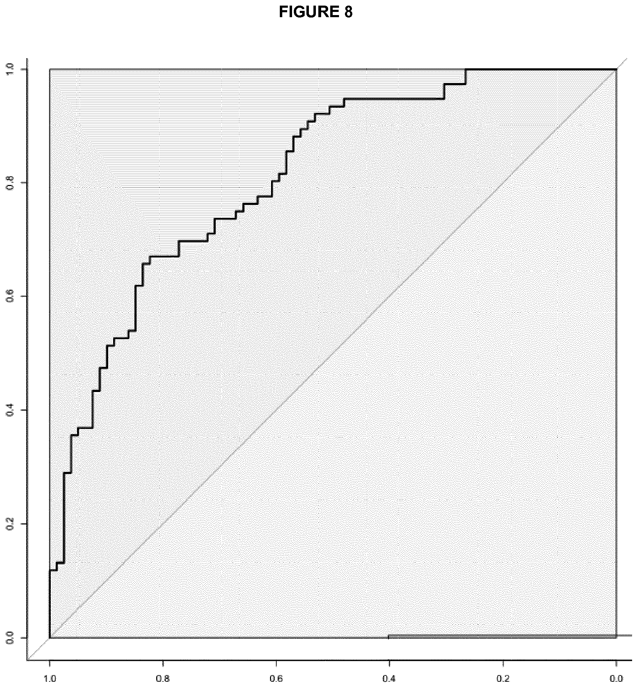

**Fig 8****.** Receiver-operating-characteristic (ROC) curve for miR-29a+ miR-15b+ miR-19b in colorectal advanced adenoma. Area Under Curve (AUC) = 0.813. X axis represents Specificity. Y axis represents Sensitivity.

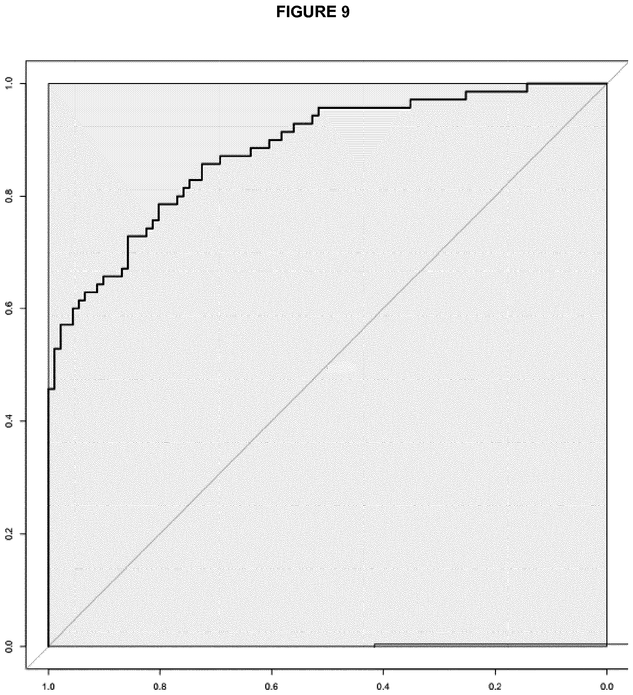

**Fig 9****.** Receiver-operating-characteristic (ROC) curve for miR-29a+ miR-15b+ miR-19a in colorectal cancer. Area Under Curve (AUC) = 0.880. X axis represents Specificity. Y axis represents Sensitivity.

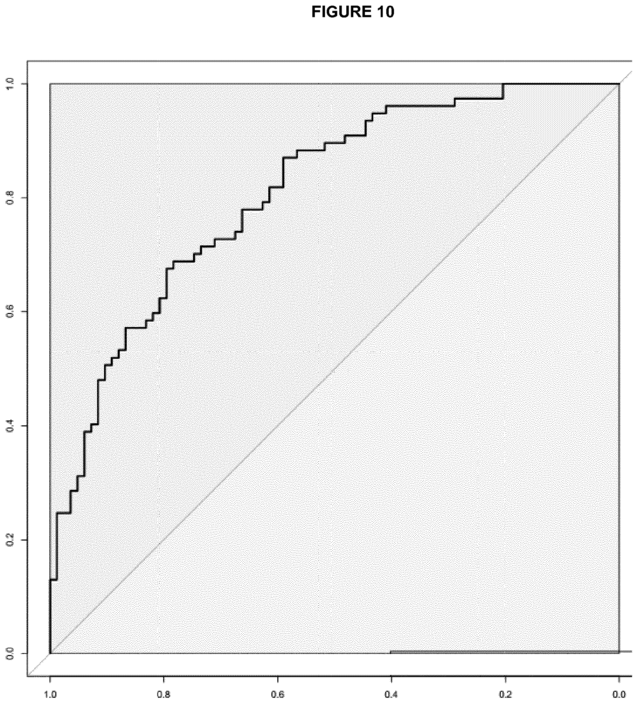

**Fig 10****.** Receiver-operating-characteristic (ROC) curve for miR-29a+ miR-15b+ miR-19a in colorectal advanced adenoma. Area Under Curve (AUC) = 0.808. X axis represents Specificity. Y axis represents Sensitivity.

### DETAILED DESCRIPTION OF THE INVENTION

### Description of the invention

The present disclosure refers to an *in vitro* method for screening or identifying subjects at risk of suffering from colorectal cancer and/or colorectal adenomas, preferably advanced colorectal adenomas, based on measuring the expression profile or level of some miRNAs which are up-regulated or over-expressed in patients suffering from said diseases. The present disclosure also refers to an *in vitro* method for obtaining useful data for the diagnosis of colorectal cancer and/or colorectal adenomas, preferably advanced colorectal adenomas, in a subject, preferably in a human subject.

In particular, the present invention is based on the discovery that miR-15b is confirmed to be significantly up-regulated in plasma samples of patients with colorectal cancer. Moreover, remarkably as shown in **Tables 6, 7** and **8,** the results provided herein also show that high circulating levels of miR-15b in plasma are significantly associated with the presence of advanced colorectal adenoma, wherein miR-15b offers the best results in comparison to the rest of the miRNAs tested herein. In fact, the results obtained with miR-15b by itself are even better than those shown for miR-29a by itself for screening for the presence of advanced colorectal adenoma or for obtaining useful data for the diagnosis of advanced colorectal adenoma in a subject, preferably in a human subject.

In addition, as shown in **Table 8,** certain specific combinations of biomarkers departing from miR-15b, such as the combination of at least (miR-15b and miR-29a), are significantly up-regulated in plasma samples of subjects suffering from colorectal cancer and advanced adenomas. In this sense, as shown in **Table 8,** the combination of at least miR-15b and miR-29a provides significantly better results in terms of AUC, sensitivity and specificity values for both detecting the presence of advanced colorectal adenoma and for detecting the presence of colorectal cancer in comparison to the use of miR-15b or miR-29a by themselves.

Consequently, a first aspect of the present disclosure refers to an *in vitro* method for screening for subjects, preferably human subjects, at risk of developing colorectal adenomas and/or colorectal cancer comprising: (a) measuring the expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) obtained from a minimally-invasive biological sample of the human subjects to be screened and (b) comparing said expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) of the human subjects to be screened with an already established expression pattern or level, wherein overexpression of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) is indicative of colorectal adenomas and/or colorectal cancer. In a preferred embodiment, the minimally-invasive biological sample obtained in the step (a) comprises: blood sample, plasma sample or serum sample. In a preferred embodiment, colorectal adenoma is advanced colorectal adenoma. In a preferred embodiment, the step (a) which comprises measuring the expression pattern or level of one microRNAs, is carried out by using a detectably labeled probe that hybridizes to a least one of the miRNAs described above.

A second aspect of the present disclosure refers to an *in vitro* method for the diagnosis of colorectal adenoma and/or colorectal cancer in a subject, preferably in a human subject, comprising: (a) measuring the expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) obtained from a minimally-invasive biological sample of the subjects, preferably human subjects, suspected of suffering from colorectal adenoma and/or colorectal cancer, (b) comparing the expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) obtained from a minimally-invasive biological sample of the subjects, preferably human subjects, suspected of suffering from colorectal adenoma and/or colorectal cancer with the expression pattern or level of a normal subject, wherein the normal subject is a healthy subject not suffering from colorectal adenoma and/or colorectal cancer, and wherein over-expression of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) is indicative of colorectal adenoma and/or colorectal cancer, and optionally (c) confirming the diagnosis by means of the examination of the bowel by any means, preferably using colonoscopy. In a preferred embodiment, the minimally-invasive biological sample obtained in the step (a) comprises: blood sample, plasma sample or serum sample. In a preferred embodiment, colorectal adenoma is advanced colorectal adenoma. In a preferred embodiment, the step (a) which comprises measuring the expression pattern or level of one microRNAs, is carried out by using a detectably labeled probe that hybridizes to a least one of the miRNAs described above.

A third aspect of the present disclosure refers to an *in vitro* method for obtaining useful data for the diagnosis of colorectal adenoma and/or colorectal cancer in subjects, preferably human subjects, comprising: (a) measuring the expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) obtained from a minimally-invasive biological sample of the subjects, preferably human subjects, suspected of suffering from colorectal cancer and/or colorectal adenoma and (b) comparing said expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) of the subjects, preferably human subjects, suspected of suffering from colorectal cancer and/or colorectal adenoma with an already established expression pattern or level, wherein overexpression of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) is indicative of colorectal adenomas and/or colorectal cancer. In a preferred embodiment, the minimally-invasive biological sample obtained in the step (a) comprises: blood sample, plasma sample or serum sample. In a preferred embodiment, colorectal adenoma is advanced colorectal adenoma. In a preferred embodiment, the step (a) which comprises measuring the expression pattern or level of one microRNAs, is carried out by using a detectably labeled probe that hybridizes to a least one of the miRNAs described above.

A fourth aspect of the present disclosure refers to the use of a kit comprising biomarker detecting reagents for determining a differential expression level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a), wherein overexpression of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) is indicative of colorectal adenoma and/or colorectal cancer, for diagnosing *in vitro* the risk for colorectal adenoma and/or colorectal cancer. In a preferred embodiment, colorectal adenoma is advanced colorectal adenoma. In a preferred embodiment, the step which comprises measuring the expression pattern or level of one microRNA, is carried out by using a detectably labeled probe that hybridizes to a least one of the miRNAs described above.

A fifth aspect of the present disclosure refers to an *in vitro* method for classifying subjects, preferably human subjects, as healthy subjects or as subjects suffering from colorectal adenoma and/or colorectal cancer comprising: (a) measuring the expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) obtained from a minimally-invasive biological sample of the subjects, preferably human subjects, to be classified and (b) comparing said expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) of the subjects, preferably human subjects, to be classified with an already established expression pattern or level, wherein overexpression of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) is indicative of colorectal adenomas and/or colorectal cancer. In a preferred embodiment, the minimally-invasive biological sample obtained in the step (a) comprises: blood sample, plasma sample or serum sample. In a preferred embodiment, colorectal adenoma is advanced colorectal adenoma. In a preferred embodiment, the step (a) which comprises measuring the expression pattern or level of one microRNAs, is carried out by using a detectably labeled probe that hybridizes to a least one of the miRNAs described above.

A sixth aspect of the present disclosure refers to a method for detecting any possible combination of at least two, three, four or five biomarkers from the following miRNAs: miR-29a, miR-15b, miR-18a, miR-19a or miR-19b. In a preferred embodiment the biomarkers detected in the present invention are: at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a).

A seventh aspect of the present disclosure refers to a method for treating subjects, preferably human subjects, suffering from colorectal adenoma and/or colorectal cancer comprising: (a) measuring *in vitro* the expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) obtained from a minimally-invasive biological sample of the subjects, preferably human subjects, to be treated, (b) comparing said expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) of the subjects, preferably human subjects, to be treated with an already established expression pattern or level, wherein overexpression of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) is indicative of colorectal adenomas and/or colorectal cancer and (c) treating the patient diagnosed with colorectal adenoma and/or colorectal cancer. In a preferred embodiment, the minimally-invasive biological sample obtained in the step (a) comprises: blood sample, plasma sample or serum sample. In a preferred embodiment, colorectal adenoma is advanced colorectal adenoma. In a preferred embodiment, the step (a) which comprises measuring the expression pattern or level of one microRNAs, is carried out by using a detectably labeled probe that hybridizes to a least one of the miRNAs described above. In a preferred embodiment the method comprises confirming the diagnosis by means of the examination of the bowel by any means, preferably using colonoscopy. Since colorectal adenoma can be seen as a precursor of colorectal cancer, because of the acknowledged adenoma-carcinoma sequence, and the notion that advanced colorectal adenomas are more likely to transition to cancer, it is well established that colorectal adenomas, and preferably colorectal advanced adenomas, should be treated, preferably, by being removed through colonoscopy (subsequent surveillance could be performed). Treatment of colorectal cancer depends on the stage at which cancer was discovered. Early stage colorectal cancer is best treated with surgery. Approximately 95% of Stage I and 65-80% of Stage II colorectal cancers are curable with surgery. Rectal cancer, however, may require additional radiation therapy to minimize the risk of recurrence. Advanced stage (Stage III and Stage IV) treatment often comprises combination of therapies, including: surgery, chemotherapy, treatment with antibodies, therapies anti-VEGF/R and radiation.

An eight aspect of the present disclosure refers to an *in vitro* method for assessing or monitoring the response to a therapy in a subject suffering from colorectal adenoma and/or colorectal cancer comprising: (a) measuring the expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) obtained from a minimally-invasive biological sample of the subjects, preferably human subjects, to be monitored and (b) comparing said expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) of the subjects, preferably human subjects, to be monitored with an already established expression pattern or level, wherein overexpression of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) is indicative of colorectal adenomas and/or colorectal cancer. A significant decrease or lack of change in the miRNA levels after the administration of said therapy in comparison with the level of the miRNAs prior to the administration of the therapy in the subject sample is indicative that the therapy administered to the subject is efficacious. A significant increase in the miRNA levels after the administration of said therapy in comparison with the level of expression of each of the miRNAs prior to the administration of the therapy in the subject sample is indicative that the therapy administered to the subject is inefficacious. In a preferred embodiment, the minimally-invasive biological sample obtained in the step (a) comprises: blood sample, plasma sample or serum sample. In a preferred embodiment, colorectal adenoma is advanced colorectal adenoma. In a preferred embodiment, the step (a) which comprises measuring the expression pattern or level of one microRNAs, is carried out by using a detectably labeled probe that hybridizes to a least one of the miRNAs described above.

A ninth aspect of the present disclosure refers to an *in vitro* method for monitoring the progression of colorectal adenoma and/or colorectal cancer in a subject comprising: (a) measuring the expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) obtained from a minimally-invasive biological sample of the subjects, preferably human subjects, to be monitored and (b) comparing said expression pattern or level of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) of the subjects, preferably human subjects, to be monitored with an already established expression pattern or level, wherein overexpression of at least (miR-15b) or of at least (miR-15b and miR-29a) or of at least (miR-15b and miR-18a), or of at least (miR-15b and miR-19b), or of at least (miR-15b and miR-19a), or of at least (miR-15b, miR-29a and miR-18a), or of at least (miR-15b, miR-19b and miR-18a), or of at least (miR-15b, miR-29a and miR-19b), or of at least (miR-15b, miR-19a and miR-18a), or of at least (miR-15b, miR-29a and miR-19a), or of at least (miR-15b, miR-19a and miR-19b), or of at least (miR-15b, miR-29a, miR-19a, miR-19b and miR-18a) is indicative of colorectal adenomas and/or colorectal cancer. A significant decrease or a lack of change in the miRNA levels in comparison with the level of expression of said miRNAs at the earlier time point is indicative that the colorectal adenoma and/or colorectal cancer shows a good progression. A significant increase in the miRNA levels in comparison with the level of expression of said miRNAs at the earlier time point is indicative that the colorectal adenoma and/or colorectal cancer shows a bad progression. In a preferred embodiment, the minimally-invasive biological sample obtained in the step (a) comprises: blood sample, plasma sample or serum sample. In a preferred embodiment, colorectal adenoma is advanced colorectal adenoma. In a preferred embodiment, the step (a) which comprises measuring the expression pattern or level of one microRNAs, is carried out by using a detectably labeled probe that hybridizes to a least one of the miRNAs described above.

A tenth aspect of the present disclosure refers to a kit comprising biomarker detecting reagents for determining a differential expression level of one or more miRNAs selected from the group consisting of: miR15b; miR-15b and miR-29a; miR-15b and miR-29a and miR-18a; miR-15b and miR-29a and miR-19a; miR-15b and miR-29a and miR-19b; miR-15b and miR-29a and miR-18a and miR-19a and miR-19b. Please note that this specific embodiment of the invention refers to a numerous clausus of miRNAs. In addition, preferably the aforesaid kit comprises reagents selected from the group consisting of all or of at least one of the following: i) specific primers to the miRNA or combinations of miRNAs as defined in claim 13 capable of producing primer-ligated miRNA sequences; ii) reverse transcribing means to produce cDNAs from the primer-ligated miRNA sequences of i); iii) means such as primers capable of amplifying the cDNAs derived from the primer-ligated miRNA sequences as defined in i); iv) means to transcribe the amplified cDNAs to produce sense target RNAs; and v) a population of miRNA antisense probes capable of detecting the sense target RNAs of iv).

An eleventh aspect of the present disclosure refers to the use of the kit of the tenth embodiment of the invention, for determining a differential expression level of at least miR15b, wherein overexpression of at least miR15b is indicative of colorectal adenomas or colorectal cancer, for screening or obtaining useful data for diagnosing *in vitro* the risk for colorectal adenomas, preferably advanced colorectal adenoma, and/or colorectal cancer.

On the other hand, it is worth mentioning that the present invention is carried out in plasma or serum samples obtained from the patients, and that it is well known in the prior art that the fact that a given biomarker is suitable for the diagnosis of a given disease departing from tissue samples, does not directly mean that the same positive result will be reproduced when the sample used for implementing the method is serum or plasma. On the other hand, it is important to emphasize that obtaining serum or plasma preparations from blood comprises several steps carried out by technicians: in the case of serum, allowing the blood to clot by leaving it undisturbed at room temperature, removing the clot by centrifuging and isolating the supernatant which is designed as serum. In the case of plasma, centrifugation is also needed. Moreover, after the centrifugation process, it is important to immediately transfer the liquid component (serum or plasma) into a clean tube. The samples are maintained at 2-8°C while handling. If the serum or plasma is not analyzed immediately, it should be apportioned into aliquots, stored, and transported at -80°C or lower. It is important to avoid freeze-thaw cycles because this is detrimental to many serum components. Samples which are hemolyzed, icteric or lipemic can invalidate certain tests.

For the purpose of the present invention, the following definitions are included below:
- The term *"screening"* is understood as the examination or testing of a group of individuals pertaining to the general population, at risk of suffering from colorectal cancer or colorectal adenoma, with the objective of discriminating healthy individuals from those who are suffering from an undiagnosed colorectal cancer or colorectal adenoma or who are at high risk of suffering from said indications.
- The term *"colorectal cancer"* is a medical condition characterized by cancer of cells of the intestinal tract below the small intestine (i.e., the large intestine (colon), including the cecum, ascending colon, transverse colon, descending colon, sigmoid colon, and rectum).
- The expression *"colorectal adenoma"* refers to adenomas of the colon, also called adenomatous polyps, which is a benign and pre-cancerous stage of the colorectal cancer but still with high risk of progression to colorectal cancer.
- The expression "advanced colorectal adenoma" refers to adenomas having a size of at least 10 mm or histologically having high grade dysplasia or a villous component higher than 20%.
- The expression *"minimally-invasive biological sample"* refers to any sample which is taken from the body of the patient without the need of using harmful instruments, other than fine needles used for taking the blood from the patient, and consequently without being harmfully for the patient. Specifically, minimally-invasive biological sample refers in the present invention to: blood, serum, or plasma samples.
- The term *"up-regulated"* or *"over-expressed"* of any of the micro-RNAs or combinations thereof described in the present invention, refers to an increase in their expression level with respect to a given "threshold value" or "cutoff value" by at least 5%, by at least 10%, by at least 15%, by at least 20%, by at least 25%, by at least 30%, by at least 35%, by at least 40%, by at least 45%, by at least 50%, by at least 55%, by at least 60%>, by at least 65%>, by at least 70%, by at least 75%, by at least 80%, by at least 85%, by at least 90%, by at least 95%, by at least 100%, by at least 110%, by at least 120%, by at least 130%, by at least 140%, by at least 150%, or more.
- The term *"threshold value"* or *"cutoff value",* when referring to the expression levels of the miRNAs described in the present invention, refers to a reference expression level indicative that a subject is likely to suffer from colorectal cancer or colorectal adenoma with a given sensitivity and specificity if the expression levels of the patient are above said threshold or cut-off or reference levels.
- The term *"comprising"* it is meant including, but not limited to, whatever follows the word "comprising". Thus, use of the term "comprising" indicates that the listed elements are required or mandatory, but that other elements are optional and may or may not be present.
- By *"consisting of'* is meant including, and limited to, whatever follows the phrase "consisting of". Thus, the phrase "consisting of" indicates that the listed elements are required or mandatory, and that no other elements may be present.
- It is also noted that the term "kit" as used herein is not limited to any specific device and includes any device suitable for working the invention such as but not limited to microarrays, bioarrays, biochips or biochip arrays.

A variety of statistical and mathematical methods for establishing the threshold or cutoff level of expression are known in the prior art. A threshold or cutoff expression level for a particular biomarker may be selected, for example, based on data from Receiver Operating Characteristic (ROC) plots, as described in the Examples and Figures of the present invention. One of skill in the art will appreciate that these threshold or cutoff expression levels can be varied, for example, by moving along the ROC plot for a particular biomarker or combinations thereof, to obtain different values for sensitivity or specificity thereby affecting overall assay performance. For example, if the objective is to have a robust diagnostic method from a clinical point of view, we should try to have a high sensitivity. However, if the goal is to have a cost-effective method we should try to get a high specificity. The best cutoff refers to the value obtained from the ROC plot for a particular biomarker that produces the best sensitivity and specificity. Sensitivity and specificity values are calculated over the range of thresholds (cutoffs). Thus, the threshold or cutoff values can be selected such that the sensitivity and/or specificity are at least about 70 %, and can be, for example, at least 75 %, at least 80 %, at least 85 %, at least 90 %, at least 95 %, at least 96 %, at least 97 %, at least 98 %, at least 99 % or at least 100% in at least 60 % of the patient population assayed, or in at least 65 %, 70 %, 75 % or 80 % of the patient population assayed.

Consequently, each of the above cited embodiments of the present invention is preferably carried out by determining the expression levels of at least the micro-RNAs previously cited in a minimally-invasive sample isolated from the subject to be diagnosed or screened, and comparing the expression levels of said micro-RNAs with predetermined threshold or cutoff values, wherein said predetermined threshold or cutoff values correspond to the expression level of said micro-RNAs which correlates with the highest specificity at a desired sensitivity in a ROC curve calculated based on the expression levels of the micro-RNAs determined in a patient population being at risk of suffering colorectal cancer or colorectal adenoma, wherein the overexpression of at least one of said micro-RNAs with respect to said predetermined cutoff value is indicative that the subject suffers from colorectal cancer or colorectal adenoma with said desired sensitivity.

The inventions illustratively described herein may suitably be practiced in the absence of any element or elements, limitation or limitations, not specifically disclosed herein. Thus, for example, the terms "comprising", "including", "containing", etc. shall be read expansively and without limitation.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non-limiting examples. In addition, where features or aspects of the invention are described in terms of groups, those skilled in the art will recognize that the invention is also thereby described in terms of any individual member or subgroup of members of the group.

### EXAMPLES

### Example 1. Population of study.

A total of 300 subjects from eight Spanish hospitals (Hospital Clinic de Barcelona, Hospital de Burgos, Hospital de Vigo, Hospital de Tenerife, Hospital de Alicante, Hospital de Donosti, Hospital de Ourense and Hospital de Zaragoza) were prospectively included in this study: 193 patients newly diagnosed with sporadic colorectal neoplasia (92 with CRC and 101 with AA) and 100 healthy individuals without personal history of any cancer and with a recent colonoscopy confirming the lack of colorectal neoplastic lesions. Patients with AA were those with adenomas having a size of at least 10 mm or histologically having high grade dysplasia or >20% villous component. The characteristics of participants are shown in **Table 1.** Blood samples were collected prior to endoscopy or surgery in all individuals.

The study was approved by the Institutional Ethics Committee of Hospital Clinic of Barcelona (approval date: 03/11/2014), and written informed consent was obtained from all participants in accordance with the Declaration of Helsinki.

**Table 1**

| | Total (n=293) | Control (n=100) | AA (n=101) | CRC (n=92) |
|---|---|---|---|---|
| Mean age (SD) | 65,7 (11,5) | 60,7(11,1) | 63,8 (9,4) | 73,1 (10,6) |
| **GENDER** | | | | |
| Male | 170 | 51 | 73 | 46 |
| Female | 123 | 49 | 28 | 46 |

| **COLORECTAL FEATURES** | | | | |
|---|---|---|---|---|
| TNM stage | | | | |
| I | - | - | - | 20 |
| II | - | - | - | 23 |
| III | - | - | - | 32 |
| IV | - | - | - | 12 |
| Unknown | - | - | - | 5 |

| Location | | | | |
|---|---|---|---|---|
| Ascending colon and cecum | - | - | - | 30 |
| Descending colon and sigma | - | - | - | 38 |
| Transverse colon | - | - | - | 6 |
| Rectum | - | - | - | 18 |

| Proximal/Distal | | | | |
|---|---|---|---|---|
| Proximal | - | - | - | 36 |
| Distal | - | - | - | 56 |

| **ADVANCED COLORECTAL ADENOMA FEATURES** | | | | |
|---|---|---|---|---|
| Size = > 10 mm | - | - | 93 | - |
| Mean size (mm) (SD) | - | - | 20,2 (11,8) | - |
| Small AA (<=15mm) | - | - | 51 | - |
| Big AA (>15mm) | - | - | 47 | - |
| No. AAs Mean (SD) | - | - | 3(3) | - |
| Stage 0 | - | - | 6 | - |

| High-grade dysplasia | | | | |
|---|---|---|---|---|
| Yes | - | - | 38 | - |
| No | - | - | 63 | - |

| Villous component | | | | |
|---|---|---|---|---|
| Yes | - | - | 41 | - |
| No | - | - | 55 | - |
| Unknown | - | - | 5 | - |

### Example 2. RNA extraction.

Ten mL of whole blood from each participant were collected in EDTA K3 containing tubes. Blood samples were placed at 4°C until plasma separation. Samples were centrifuged at 1,600 x g for 10 min at 4°C to spin down blood cells, and plasma was transferred into new tubes, followed by further centrifugation at 16,000 x g for 10 minutes at 4°C to completely remove cellular components. Plasma was then aliquoted and stored at -80°C until use. miRNA isolation was performed using mirVana^{™} PARIS^{™} kit (Ambion by Life Technologies).

The miRNAs were extracted from all plasma samples, retro-transcribed, pre-amplified and analyzed by Real-Time quantitative PCR. For each RNA sample, 3 control miRNAs (cel-miR-39-3p as "spike-in", and hsa-miR-1228-3p) and 5 candidate miRNAs (hsa-miR-15b-5p, hsa-miR-18a-5p, hsa-miR-29a-3p, hsa-miR-19a-3p and hsa-miR-19b-3p) were analyzed separately and in combination. Each miRNA in each sample was assessed in triplicate and mean Ct values were normalized in order to obtain -deltaCT values (DCt).

MiRNA singleplex Retrotranscription protocol (RT) was performed using RT primers from TaqMan^{®} MicroRNA Assays. (P/N: 4427975. Assay ID: specific for each miRNA-Life Technologies and using TaqMan^{®} MicroRNA Reverse Transcription Kit. Life Technologies): Cel-miR-39-3p (Assay ID: 000200 Exogenous control), Hsa-miR-1228-3p (Assay ID: 002919 Endogenous control), Hsa-miR-15b-5p (Assay ID: 000390), Hsa-miR-18a-5p (Assay ID: 002422), Hsa-miR-29a-3p (Assay ID: 002112), Hsa-miR-19a-3p *(Assay ID: 000395)* and Hsa-miR-19b-3p *(Assay ID: 000396).*

RT protocol was performed in Veriti^{®} 96 well Thermal Cycler P/N (product number): 4375786 (Life Technologies). MiRNA singleplex Preamplification protocol (Preamp) was performed for all miRNAs analyzed. Real Time primers from TaqMan^{®} MicroRNA Assays (P/N Assay ID: specific for each miRNA. Life Technologies as previously described) were used. TaqMan^{®} Preamp Master Mix was used (Life Technologies). PreAmplification analyses were performed in Veriti^{®} 96 well Thermal Cycler P/N: 4375786 (Life Technologies.) Real Time PCR protocol for miRNA expression analysis were performed using Real Time primer 20X from TaqMan^{®} MicroRNA Assays (P/N Assay ID: specific for each miRNA-Life Technologies as previously described). Expression analyses were performed in a ViiA^{™} 7 Real-Time PCR System (P/N: 4375786-Life technologies.) Ct values were calculated from automatic threshold. No template controls showed no amplification. Three technical replicates were included for each point of qPCR. Relative expression levels of selected miRNAs were calculated for each sample as DCt values [DCt = Ct of target miRNA - Ct of internal control miRNA].

### Example 3. Statistical analysis.

Differences in the miRNA expression levels (-DCt values) between different groups of patients were explored using multivariate logistic regression analysis adjusted by age, gender and site computing the odds ratio and intervals and their corresponding p values. ROC analysis plots and derived cut-points, as well as overall discriminative accuracy parameters, have been computed using DiagnosisMed R-package considering each miRNA expression as a continuous variable. Sensitivity (Sn), specificity (Sp), were calculated for several cut-points (the optimum cut-point associated with the minimum distance between the ROC curve and upper left corner, the cut-point associated with 80% Sn, with 85% Sn and with 90% Sn) (see **Table 2** for colorectal cancer and **Table 3** for advanced colorectal adenoma).

**Table 2**

| **Colorectal Cancer** | **Critera** | **Cut-off** | **Sn** | **Sp** |
|---|---|---|---|---|
| **miR-19a+miR-19b+miR-15b** | | | | |
| AUC=0.845 | Min.ROC distance | 0.530 | 70 | 86 |
| AUC=0.845 | Max.Youden Index | 0.618 | 64 | 94 |

| miR-19a+miR-29a+miR-15b | | | | |
|---|---|---|---|---|
| AUC= 0.880 | Min.ROC distance | 0,466 | 76 | 88 |
| AUC= 0.880 | Max.Youden Index | 0,466 | 76 | 88 |
| AUC= 0.880 | Sn=Sp | 0,430 | 79 | 80 |
| AUC= 0.880 | Sn 80% | 0,377 | 81 | 75 |

| **miR-19a+miR-15b+miR-18a** | | | | |
|---|---|---|---|---|
| AUC=0.842 | Max.Youden Index | 0.580 | 66 | 93 |

| **miR-19b+miR-29a+miR-15b** | | | | |
|---|---|---|---|---|
| AUC=0.876 | Min.ROC distance | 0,524 | 74 | 89 |
| AUC=0.876 | Max.Youden Index | 0,524 | 74 | 89 |
| AUC=0.876 | Sn=Sp | 0,415 | 77 | 77 |
| AUC=0.876 | Sn=80% | 0,396 | 80 | 75 |

| **miR-19b+miR-15b+miR-18a** | | | | |
|---|---|---|---|---|
| AUC=0.845 | Max.Youden Index | 0.543 | 67 | 92 |

| **miR-29a+miR-15b+miR-18a** | | | | |
|---|---|---|---|---|
| AUC= 0.880 | Min.ROC distance | 0,504 | 75 | 87 |
| AUC= 0.880 | Max.Youden Index | 0,549 | 72 | 90 |
| AUC= 0.880 | Sp 70% | 0,362 | 80 | 71 |
| AUC= 0.880 | Sn=80% | 0,333 | 81 | 69 |

| **miR-19a+miR-15b** | | | | |
|---|---|---|---|---|
| AUC=0.838 | Min.ROC distance | 0.518 | 70 | 88 |
| AUC=0.838 | Max.Youden Index | 0.621 | 62 | 95 |

| **miR-19b+miR-15b** | | | | |
|---|---|---|---|---|
| AUC=0.839 | Min.ROC distance | 0.518 | 70 | 88 |
| AUC=0.839 | Max.Youden Index | 0.640 | 64 | 95 |

| **miR-29a+miR-15b** | | | | |
|---|---|---|---|---|
| AUC= 0.878 | Min.ROC distance | 0,478 | 75 | 88 |
| AUC= 0.878 | Max.Youden Index | 0,478 | 75 | 88 |
| AUC= 0.878 | Sn=Sp | 0,435 | 78 | 79 |

| **miR-15b+miR-18a** | | | | |
|---|---|---|---|---|
| AUC=0.836 | Max.Youden Index | 0.590 | 64 | 93 |

| **miR-15b** | | | | |
|---|---|---|---|---|
| AUC= 0.833 | Min.ROC distance | 0,514 | 68 | 86 |
| AUC= 0.833 | Max.Youden Index | 0.621 | 63 | 95 |
| AUC= 0.833 | Sn 80% | 0,301 | 80 | 58 |
| AUC= 0.833 | Sn 85% | 0,284 | 85 | 58 |
| AUC= 0.833 | Sn 90% | 0,215 | 90 | 44 |

**Table 3**

| **Advanced Colorectal Adenoma** | **Criteria** | **Cut-off** | **Sn** | **Sp** |
|---|---|---|---|---|
| **miR-19a+miR-19b+miR-15b** | | | | |
| AUC= 0.799 | Min.ROC distance | 0.510 | 67 | 75 |
| AUC= 0.799 | Max.Youden Index | 0.346 | 87 | 58 |

| **miR-19a+miR-29a+miR-15b** | | | | |
|---|---|---|---|---|
| AUC= 0.808 | Min.ROC distance | 0,523 | 71 | 78 |
| AUC= 0.808 | Max.Youden Index | 0,523 | 71 | 78 |
| AUC= 0.808 | Sn=Sp | 0,472 | 71 | 71 |

| **miR-19a+miR-15b+miR-18a** | | | | |
|---|---|---|---|---|
| AUC= 0.803 | Max.Youden Index | 0.290 | 91 | 52 |

| **miR-19b+miR-29a+miR-15b** | | | | |
|---|---|---|---|---|
| AUC= 0.813 | Min.ROC distance | 0.574 | 67 | 82 |
| AUC= 0.813 | Max.Youden Index | 0.574 | 66 | 82 |
| AUC= 0.813 | Sn=Sp | 0.484 | 71 | 71 |

| **miR-19b+miR-15b+miR-18a** | | | | |
|---|---|---|---|---|
| AUC= 0.798 | Max.Youden Index | 0.480 | 69 | 75 |

| **miR-29a+miR-15b+miR-18a** | | | | |
|---|---|---|---|---|
| AUC= 0.812 | Min.ROC distance | 0,517 | 74 | 73 |
| AUC= 0.812 | Max.Youden Index | 0.599 | 62 | 86 |

| **miR-19a+miR-15b** | | | | |
|---|---|---|---|---|
| AUC= 0.795 | Min.ROC distance | 0.448 | 72 | 71 |
| AUC= 0.795 | Max.Youden Index | 0.323 | 90 | 55 |

| **miR-19b+miR-15b** | | | | |
|---|---|---|---|---|
| AUC= 0.798 | Min.ROC distance | 0.483 | 68 | 74 |
| AUC= 0.798 | Max.Youden Index | 0.332 | 88 | 54 |

| **miR-29a+miR-15b** | | | | |
|---|---|---|---|---|
| AUC= 0.811 | Min.ROC distance | 0,469 | 74 | 73 |
| AUC= 0.811 | Max.Youden Index | 0,469 | 74 | 73 |

| **miR-15b+miR-18a** | | | | |
|---|---|---|---|---|
| AUC= 0.805 | Max.Youden Index | 0.468 | 74 | 71 |

| **miR-15b** | | | | |
|---|---|---|---|---|
| AUC= 0.798 | Min.ROC distance | 0,438 | 76 | 67 |
| AUC= 0.798 | Max.Youden Index | 0.314 | 90 | 55 |
| AUC= 0.798 | Sn 80% | 0,381 | 81 | 63 |
| AUC= 0.798 | Sn 85% | 0,351 | 86 | 59 |
| AUC= 0.798 | Sn 90% | 0,314 | 90 | 55 |

With all Ct values from all samples obtained from the Real-Time PCR we have used different normalization transformations in order to obtain -DCt. We have also selected normalization by multiple housekeeping miRNAs (cel-miR-39, hsa-miR-1228) instead of one. Normalization factor based on expression levels of the housekeeping miRNAs have been calculated by using geometric mean (http://genomebiology.com/2002/3/7/research/0034) of the different housekeeping combinations. All 3 housekeeping miRNAs (cel-miR-39, hsa-miR-1228) showed good Ct levels in all samples and showed high stability between samples, with no differences in their expression between groups.

Initially we used different normalization schemes (normalization using cel-miR-39; normalization using hsa-miR-1228, normalization using geometric mean of cel-mir-39, and hsa-miR-1228, normalization using geometric mean of cel-miR-39, and normalization using geometric mean of cel-miR-39 and hsa-miR-1228).

Although all six normalization methods gave very similar results, the most accurate was normalization using geometric mean of cel-miR-39 and hsa-miR-1228.

### Example 4. Comparison of patients with colorectal cancer versus control individuals.

Before analyzing results we have excluded some Ct values from the analysis for being above the maximum Ct value of the linearity range (obtained from our previous analytical validation).

The comparison of colorectal cancer patients versus control individuals was also carried out. Logistic regression results: Odds ratio (OR), 95% confidence interval (CI) and the corresponding p-value from logistic regression are shown for each individual miRNA in the model adjusted by age and gender. The "n" means the number of samples that have been finally analyzed for each miRNA (see **Table 4).**

**Table 4**

| **CRC vs Control** | | | | | |
|---|---|---|---|---|---|
| **Individual miRNA** | **n** | **OR** | **Cl low** | **Cl high** | **p-value** |
| **miR-29a** | **187** | **1,80** | **2,36** | **1,37** | **0,00002** |
| **miR-15b** | **171** | **1,61** | **2,17** | **1,20** | **0,00167** |
| miR-18a | 183 | 1,26 | 1,60 | 0,99 | 0,06364 |
| miR-19a | 187 | 1,06 | 1,32 | 0,85 | 0,63336 |
| miR-19b | 190 | 0,96 | 1,20 | 0,77 | 0,74196 |

In order to correct any possible influence of the sample origin, logistic regression has also been adjusted by hospital of origin. In this analysis, only hospitals that had samples in the different groups have been included (see **Table 5**). For this reason the "n" is different from the table shown above **(Table 4).**

**Table 5**

| **CRC vs Control** | | | | | |
|---|---|---|---|---|---|
| **Individual miRNA** | **n** | **OR** | **CI low** | **CI high** | **p-value** |
| **miR-29a** | **173** | **1,94** | **2,68** | **1,40** | **0,0001** |
| **miR-15b** | **157** | **1,53** | **2,16** | **1,08** | **0,0163** |
| **miR-18a** | **169** | **1,32** | **1,73** | **1,00** | **0,0497** |
| miR-19a | 173 | 1,15 | 1,48 | 0,89 | 0,2956 |
| miR-19b | 176 | 1,03 | 1,35 | 0,78 | 0,8476 |

Interestingly, miR-18a and miR-15b were confirmed to be significantly up-regulated in patients with colorectal cancer.

### Example 5. Comparison of patients with advanced colorectal adenoma versus control individuals.

The comparison of advanced colorectal adenoma patients versus control individuals was also carried out. Logistic regression results: Odds ratio (OR), 95% confidence interval (CI) and the corresponding p-value from logistic regression are shown in **Table 6** for each miRNA in the model adjusted by age and gender. The "n" means the number of samples that have been analyzed for each miRNA.

**Table 6**

| **AA vs Control** | | | | | |
|---|---|---|---|---|---|
| **Individual miRNA** | **n** | **OR** | **CI low** | **CI high** | **p-value** |
| **miR-15b** | **183** | **1,78** | **2,33** | **1,37** | **0,00002** |
| **miR-29a** | **195** | **1,37** | **1,69** | **1,12** | **0,00235** |
| **miR-18a** | **194** | **1,27** | **1,57** | **1,03** | **0,02327** |
| miR-19a | 197 | 1,10 | 1,33 | 0,91 | 0,32414 |
| miR-19b | 201 | 1,02 | 1,24 | 0,84 | 0,85322 |

In order to correct any possible influence of sample origin, logistic regression has also been adjusted by hospital of origin. In this analysis, only hospitals that had samples in the different groups have been included (see **Table 7**)**.** For this reason the "n" is different from the **Table 6** shown above.

**Table 7**

| **AA vs Control** | | | | | |
|---|---|---|---|---|---|
| **Individual miRNA** | **n** | **OR** | **CI low** | **CI high** | **p-value** |
| **miR-15b** | **169** | **1,94** | **2,66** | **1,41** | **0,00004** |
| **miR-29a** | **181** | **1,33** | **1,66** | **1,07** | **0,01089** |
| **miR-18a** | **180** | **1,33** | **1,70** | **1,04** | **0,02413** |
| miR-19a | 183 | 1,15 | 1,42 | 0,94 | 0,17758 |
| miR-19b | 187 | 1,10 | 1,42 | 0,85 | 0,48836 |

Results show that high circulating levels of miR-15b and miR-18a are significantly associated with the presence of advanced colorectal adenoma, but miR-15b offering the best results.

### Example 6. miRNA signatures for colorectal cancer and advanced colorectal adenoma detection versus control population.

The next step was to analyze any possible combinations of at least two of the five miRNAs to test the best signature to discriminate between colorectal cancer and/or advanced colorectal adenoma vs control individuals.

All possible combinations among miR-19a, miR-19b, miR-29a, miR-15b and miR-18a have been assessed and those models showing the highest AUC (= or >0.88) for colorectal cancer vs Controls and the highest AUC (>0.80) for advanced colorectal adenoma vs Controls when adjusted by age, gender and hospital, have been selected. It is worth pointing out all the best signatures for the diagnosis of colorectal cancer and/or advanced colorectal adenoma comprises at least miR-15b. Consequently, we tested the differences between the AUC, Sensitivity (Sn) and Specificity (Sp) comparing the following signature: miR-15b vs miR-29a vs miR-15b+miR-29a vs miR-15b+miR-29a+miR-18a vs miR-15b+miR-29a+miR-19a vs miR-15b+miR-29a+miR-19b. Results are shown in **Table** 8 below (*Min.Roc distance **Max.Youden Index).

**Table 8**

| **CRC** | | | | **AA** | | | |
|---|---|---|---|---|---|---|---|
| **AUC** | **Criteria** | **Sn** | **Sp** | **AUC** | **Criteria** | **Sn** | **Sp** |
| **miR-29a** | | | | | | | |
| 0.87 | * | 73 | 82 | 0.73 | * | 76 | 61 |

| **miR-15b** | | | | | | | |
|---|---|---|---|---|---|---|---|
| 0.833 | * | 68 | 86 | 0.798 | * | 76 | 67 |

| **miR-29a+miR-15b** | | | | | | | |
|---|---|---|---|---|---|---|---|
| 0.878 | * | 75 | 88 | 0.811 | * | 74 | 73 |
| 0.878 | ** | 75 | 88 | 0.811 | ** | 74 | 73 |

| **miR-29a+miR-15b+miR-19b** | | | | | | | |
|---|---|---|---|---|---|---|---|
| 0.876 | * | 74 | 89 | 0.813 | * | 67 | 82 |
| 0.876 | ** | 74 | 89 | 0.813 | ** | 67 | 82 |

| **miR-29a+miR-15b+miR-18a** | | | | | | | |
|---|---|---|---|---|---|---|---|
| 0.880 | * | 75 | 87 | 0.812 | * | 74 | 73 |
| 0.880 | ** | 72 | 90 | 0.812 | ** | 63 | 86 |

| **miR-29a+miR-15b+miR-19a** | | | | | | | |
|---|---|---|---|---|---|---|---|
| 0.880 | * | 76 | 88 | 0.808 | * | 71 | 78 |
| 0.880 | ** | 76 | 88 | 0.808 | ** | 71 | 78 |

ROC curve parameters for the signature comprising miR-15b+miR-29a (area under curve (AUC) and 95% confidence interval (CI) are shown for all CRC cases as well as for different tumor stages (I/II and III/IV) and locations (right and left, with respect to the splenic flexure). In addition, we have analyzed AA taking into account AA > 10 mm vs AA< 10mm (see **Table 9** for CRC and **Table 10** for AA). (*Min.Roc distance **Max.Youden Index).

**Table 9**

| **AUC** | **Criteria** | **Sn** | **Sp** |
|---|---|---|---|
| **CRC vs Control** | | | |
| 0.88 (0.82-0.93) | * | 75 | 88 |
| 0.88 (0.82-0.93) | ** | 75 | 88 |

| **CRC (I/II) vs Control** | | | |
|---|---|---|---|
| 0.89 (0.83-0.96) | * | 78 | 88 |
| 0.89 (0.83-0.96) | ** | 78 | 88 |

| **CRC (III/IV) vs Control** | | | |
|---|---|---|---|
| 0.89 (0.82-0.96) | * | 86 | 77 |
| 0.89 (0.82-0.96) | ** | 86 | 77 |

| **CRC (Distal/Left) vs Control** | | | |
|---|---|---|---|
| 0.88 (0.82-0.94) | * | 83 | 76 |
| 0.88 (0.82-0.94) | ** | 73 | 87 |

| **CRC (Proximal/Right) vs Control** | | | |
|---|---|---|---|
| 0.88 (0.80-0.96) | * | 71 | 94 |
| 0.88 (0.80-0.96) | ** | 71 | 94 |

**Table 10**

| **AUC** | **Criteria** | **Sn** | **Sp** |
|---|---|---|---|
| **AA vs Control** | | | |
| 0.81 (0.75-0.87) | * | 74 | 73 |
| 0.81 (0.75-0.87) | ** | 74 | 73 |

| **Small AA vs Control** | | | |
|---|---|---|---|
| 0.83 (0.75-0.91) | * | 77 | 77 |
| 0.83 (0.75-0.91) | ** | 64 | 92 |

| **Big AA vs Control** | | | |
|---|---|---|---|
| 0.84 (0.77-0.91) | * | 74 | 80 |
| 0.84 (0.77-0.91) | ** | 85 | 70 |

## Claims

1. *In vitro* method for the diagnosis of subjects at risk of developing advanced colorectal adenomas comprising: (a) measuring the expression pattern or level of at least miR-15b and miR-29a obtained from a minimally-invasive biological sample of the human subjects to be diagnosed selected from the list consisting of: a plasma sample, blood sample or serum sample; and (b) comparing said expression pattern or level of at least miR-15b and miR-29a of the human subjects to be diagnosed with an already established expression pattern or level, wherein the overexpression of at least miR15b and miR-29a is indicative of advanced colorectal adenomas.

2. *In vitro* method for the diagnosis subjects at risk of developing advanced colorectal adenomas, according to the claim 1, comprising: (a) measuring the expression pattern or level of at least miR-15b and miR-29a and miR-18a, or of at least miR-15b and miR-29a and miR-19a, or of at least miR-15b and miR-29a and miR-19b, or of at least miR-15b and miR-29a and miR-18a and miR-19a and miR-19b, obtained from a minimally-invasive biological sample of the human subjects to be diagnosed selected from the group consisting of: blood sample, plasma sample or serum sample; and (b) comparing said expression pattern or level of at least miR-15b and miR-29a, or of at least miR-15b and miR-29a and miR-18a, or of at least miR-15b and miR-29a and miR-19a, or of at least miR-15b and miR-29a and miR-19b, or of at least miR-15b and miR-29a and miR-18a and miR-19a and miR-19b, of the subjects to be diagnosed with an already established expression pattern or level, wherein overexpression of at least miR-15b and miR-29a, or of at least miR-15b and miR-29a and miR-18a, or of at least miR-15b and miR-29a and miR-19a, or of at least miR-15b and miR-29a and miR-19b, or of at least miR-15b and miR-29a and miR-18a and miR-19a and miR-19b is indicative of advanced colorectal adenomas.

3. *In vitro* method for the diagnosis of a subject at risk of developing advanced colorectal adenoma, according to any of the claims 1 or 2, comprising the steps a) and b) of any of the claims 1 or 2, and (c) confirming the presence of advanced colorectal adenomas by means of the examination of the bowel by any means, preferably using colonoscopy.

4. Use of a kit comprising reagents for determining a differential expression level of at least miR-15b and miR-29a, or of at least miR-15b and miR-29a and miR-18a, or of at least miR-15b and miR-29a and miR-19a or of at least miR-15b and miR-29a and miR-19b or of at least miR-15b and miR-29a and miR-18a and miR-19a and miR-19b for diagnosing *in vitro* the risk for colorectal advanced adenomas, in a plasma, blood or serum sample.

5. A kit consisting of detecting reagents for determining the expression level of miR-15b and miR-29a in a biological sample of the human subjects selected from the list consisting of: a plasma sample, blood sample or serum sample, wherein said reagents are selected from specific primers to miR-15b and miR-29a and detectably labelled probes that hybridize to miR-15b and miR-29a.

## Patentansprüche

1. *In-vitro*-Verfahren für die Diagnose von Individuen, welche gefährdet sind, fortgeschrittene kolorektale Adenome zu entwickeln, umfassend: (a) das Messen des Expressionsmusters oder -niveaus von mindestens miR-15b und miR-29a, welches aus einer minimalinvasiven biologischen Probe der zu diagnostizierenden menschlichen Individuen erhalten wird, ausgewählt aus der Liste bestehend aus: einer Plasmaprobe, Blutprobe oder Serumprobe; und (b) das Vergleichen des genannten Expressionsmusters oder-niveaus von mindestens miR-15b und miR-29a der zu diagnostizierenden menschlichen Individuen mit einem bereits festgestellten Expressionsmuster oder -niveau, wobei die Überexpression von mindestens miR-15b und miR-29a auf fortgeschrittene kolorektale Adenome schließen lässt.

2. *In-vitro*-Verfahren für die Diagnose von Individuen, welche gefährdet sind, fortgeschrittene kolorektale Adenome zu entwickeln, nach Anspruch 1, umfassend: (a) das Messen des Expressionsmusters oder -niveaus von mindestens miR-15b und miR-29a und miR-18a, oder von mindestens miR-15b und miR-29a und miR-19a, oder von mindestens miR-15b und miR-29a und miR-19b, oder von mindestens miR-15b und miR-29a und miR-18a und miR-19a und miR-19b, welches aus einer minimalinvasiven biologischen Probe der zu diagnostizierenden menschlichen Individuen erhalten wird, ausgewählt aus der Gruppe bestehend aus: Blutprobe, Plasmaprobe oder Serumprobe; und (b) das Vergleichen des genannten Expressionsmusters oder -niveaus von mindestens miR-15b und miR-29a, oder von mindestens miR-15b und miR-29a und miR-18a, oder von mindestens miR-15b und miR-29a und miR-19a, oder von mindestens miR-15b und miR-29a und miR-19b, oder von mindestens miR-15b und miR-29a und miR-18a und miR-19a und miR-19b, der zu diagnostizierenden Individuen mit einem bereits festgestellten Expressionsmuster oder -niveau, wobei die Überexpression von mindestens miR-15b und miR-29a, oder von mindestens miR-15b und miR-29a und miR-18a, oder von mindestens miR-15b und miR-29a und miR-19a, oder von mindestens miR-15b und miR-29a und miR-19b, oder von mindestens miR-15b und miR-29a und miR-18a und miR-19a und miR-19b auf fortgeschrittene kolorektale Adenome schließen lässt.

3. *In-vitro*-Verfahren für die Diagnose von einem Individuum, welches gefährdet ist, fortgeschrittenes kolorektales Adenom zu entwickeln, nach einem der Ansprüche 1 oder 2, umfassend die Schritte a) und b) nach einem der Ansprüche 1 oder 2, und (c) das Bestätigen der Anwesenheit von fortgeschrittenen kolorektalen Adenomen mittels der Untersuchung des Darmes durch jedes Mittel, vorzugsweise unter Verwendung von Koloskopie.

4. Verwendung eines Kits umfassend Reagenzien zur Bestimmung eines differentiellen Expressionsniveaus von mindestens miR-15b und miR-29a, oder von mindestens miR-15b und miR-29a und miR-18a, oder von mindestens miR-15b und miR-29a und miR-19a oder von mindestens miR-15b und miR-29a und miR-19b oder von mindestens miR-15b und miR-29a und miR-18a und miR-19a und miR-19b für die *In-vitro-*Diagnose des Risikos von fortgeschrittenen kolorektalen Adenomen, in einer Plasma-, Blut- oder Serumprobe.

5. Kit bestehend aus Detektionsreagenzien zur Bestimmung des Expressionsniveaus von miR-15b und miR-29a in einer biologischen Probe des menschlichen Individuums ausgewählt aus der Liste bestehend aus: einer Plasmaprobe, Blutprobe oder Serumprobe, wobei die genannten Reagenzien aus spezifischem Primern zu miR-15b und miR-29a und detektierbar markierten Sonden, welche mit miR-15b und miR-29a hybridisieren, ausgewählt werden.

## Revendications

1. Procédé *in vitro* pour le diagnostic de sujets à risque de développer des adénomes colorectaux avancés qui comprend : (a) le mesure du niveau ou patron d'expression d'au moins miR-15b et miR-29a obtenu à partir d'un échantillon biologique minimalement invasif des sujets humains qui vont être diagnostiqués sélectionné de la liste qui consiste en : un échantillon de plasma, échantillon de sang ou échantillon de sérum ; et (b) la comparaison dudit niveau ou patron d'expression d'au moins miR-15b et miR-29a des sujets humains qui vont être diagnostiqués avec un niveau ou un patron d'expression déjà établi, dans lequel la surexpression d'au moins miR-15b et miR-29a est indicative d'adénomes colorectaux avancés.

2. Procédé *in vitro* pour le diagnostic des sujets à risque de développer des adénomes colorectaux avancés selon la revendication 1, qui comprend : (a) la mesure du niveau ou patron d'expression d'au moins miR-15b et miR-29a et miR-18a, ou d'au moins miR-15b et miR-29a et miR-19a, ou d'au moins miR-15b et miR-29a et miR-19b, ou d'au moins miR-15b et miR-29a et miR-18a et miR-19a et miR-19b, obtenu à partir d'un échantillon biologique minimalement invasif des sujets humains qui vont être diagnostiqués, sélectionné du groupe qui consiste en : échantillon de sang, échantillon de plasma ou échantillon de sérum ; et (b) la comparaison dudit niveau ou patron d'expression d'au moins miR-15b et miR-29a, ou d'au moins miR-15b et miR-29a et miR-18a, ou d'au moins miR-15b et miR-29a et miR-19a, ou d'au moins miR-15b et miR-29a et miR-19b, ou d'au moins miR-15b et miR-29a et miR-18a et miR-19a et miR-19b, des sujets qui vont être diagnostiqués avec un niveau ou patron d'expression déjà établi, dans lequel la surexpression d'au moins miR-15b et miR-29a, ou d'au moins miR-15b et miR-29a et miR-18a, ou d'au moins miR-15b et miR-29a et miR-19a, ou d'au moins miR-15b et miR-29a et miR-19b, ou d'au moins miR-15b et miR-29a et miR-18a et miR-19a et miR-19b est indicative d'adénomes colorectaux avancés.

3. Procédé *in vitro* pour le diagnostic d'un sujet à risque de développer un adénome colorectal avancé selon l'une quelconque des revendications 1 ou 2, qui comprend les étapes a) et b) selon l'une quelconque des revendications 1 ou 2, et (c) confirmation de la présence d'adénomes colorectaux avancés au moyen l'exploration de l'intestin partout moyen, préférablement en utilisant la colonoscopie.

4. Utilisation d'un kit qui comprend des réactifs pour déterminer un niveau d'expression différentiel d'au moins miR-15b et miR-29a, ou d'au moins miR-15b et miR-29a et miR-18a, ou d'au moins miR-15b et miR-29a et miR-19a ou d'au moins miR-15b et miR-29a et miR-19b ou d'au moins miR-15b et miR-29a et miR-18a et miR-19a et miR-19b pour diagnostiquer *in vitro* le risque d'adénomes colorectaux avancés, dans un échantillon de plasma, de sang ou de sérum.

5. Kit qui consiste en réactifs de détection pour déterminer le niveau d'expression de miR-15b et miR-29a dans un échantillon biologique des sujets humains sélectionné de la liste qui consiste en : un échantillon de plasma, échantillon de sang ou échantillon de sérum, dans lequel lesdits réactifs sont sélectionnés à partir d'amorces spécifiques à miR-15b et à miR-29a et de sondes étiquetées de manière détectable qui s'hybrident avec miR-15b et miR-29a.
